**Europäisches Patentamt**

**European Patent Office**

**Office européen des brevets**

(11) Veröffentlichungsnummer: **0 325 120 B1**

(12) **EUROPÄISCHE PATENTSCHRIFT**

(45) Veröffentlichungstag der Patentschrift: **28.04.93**

(51) Int. Cl.⁵: **H05G 1/26**, H05G 1/36, H05G 1/46, A61B 6/00

(21) Anmeldenummer: **89100182.8**

(22) Anmeldetag: **05.01.89**

(54) **Röntgendiagnostikanlage.**

(30) Priorität: **18.01.88 DE 3801210**

(43) Veröffentlichungstag der Anmeldung:
**26.07.89 Patentblatt 89/30**

(45) Bekanntmachung des Hinweises auf die Patenterteilung:
**28.04.93 Patentblatt 93/17**

(84) Benannte Vertragsstaaten:
**DE FR**

(56) Entgegenhaltungen:
**EP-A- 0 063 644**
**EP-A- 0 200 272**
**EP-A- 0 218 367**
**WO-A-87/01555**
**DE-A- 3 619 863**

(73) Patentinhaber: **SIEMENS AKTIENGESELL-SCHAFT**
**Wittelsbacherplatz 2**
**W-8000 München 2(DE)**

(72) Erfinder: **Aichinger, Horst, Dr.**
**Unterfarrnbacher Strasse 32**
**W-8510 Fürth(DE)**
Erfinder: **Köhler, Karlheinz**
**Haydnstrasse 90**
**W-8522 Herzogenaurach(DE)**

**Beschreibung**

Die Erfindung betrifft eine Röntgendiagnostikanlage für Mammographieaufnahmen, die einen Rechner aufweist.

Zur Abschätzung des Strahlenrisikos ist die Kenntnis der Strahlenexposition der Patientin bei der Anfertigung von Röntgenaufnahmen von Interesse. Außer von den vorgegebenen gerätespezifischen Einflußgrößen hängt die Strahlenexposition von den Aufnahmedaten (Röhrenspannung, vom Belichtungsautomaten geschaltetes Röhrenstrom-Zeit-Produkt, Filter), der Empfindlichkeit des bildgebenden Systems (Film-Folien-System) und der Objektdicke ab. Die für die Abschätzung des Strahlenrisikos relevante Dosisgröße ist die mittlere Parenchymdosis. Sie kann - bei bekannter Empfindlichkeit des bildgebenden Systems und hierdurch festgelegter Austrittsdosis - durch Messung der Eintrittsdosis ermittelt werden. Die gleichzeitige Messung der Eintrittsdosis bei der Anfertigung von Röntgenaufnahmen mit Patienten ist in der Regel nicht möglich. Wird jedoch eine Kalibrierung der Röntgenanlage mit gewebeäquivalenten Phantomen durchgeführt, ist eine Abschätzung der mittleren Parenchymdosis durch Messung des geschalteten mAs-Produktes möglich.

Aus WO-A-8 701 555 ist eine Röntgendiagnostikanlage für Mammographieaufnahmen bekannt, die einen Rechner zur Berechnung und Regelung der Bestrahlungsstärke aufweist.

Der Erfindung liegt die Aufgabe zugrunde, eine Röntgendiagnostikanlage für Mammographieaufnahmen zu schaffen, bei der eine automatische Bestimmung der Parenchymdosis ohne Einschaltung des Untersuchungspersonals erfolgt.

Diese Aufgabe ist erfindungsgemäß dadurch gelöst, daß der Rechner dazu angepaßt ist, aus den jeweiligen Aufnahmewerten entsprechenden, ihm zugeführten, elektrischen Signalen die mittlere Parenchymdosis nach folgender Formel zu berechnen:

$$\overline{D} = \frac{f \cdot J_E}{a \cdot (d - 2\,d_F)} \cdot \left[ e^{-a \cdot d_F} - e^{-a(d - d_F)} \right]$$

mit
f = 1,03 (Umrechnungsfaktor)
d = Objektdicke in cm
$d_F$ = Dicke des Fettgewebes
$d_F$ = 0,5 cm für d ≤ 5,0 cm
$d_F$ = 1,0 cm für d > 5,0 cm

$J_E = k \cdot (I \cdot t)$

I = Röntgenröhrenstrom
t = Aufnahmezeit
k = Kalibrierfaktor

$$a = \frac{1}{d} \cdot \ln \frac{J_E}{J_A}$$

$J_A$ = Austrittsdosis

Die mit Hilfe des Rechners berechnete mittlere Parenchymdosis kann über einen Drucker zur Protokollierung ausgegeben werden. Die Berechnung ist möglich, weil alle zur Berechnung erforderlichen dosisrelevanten Daten nach erfolgter Aufnahme in digitaler Form vorliegen. Die zur Ermittlung der Eintrittsdosis $J_E$ erforderlichen Kalibrierfaktoren k können in einem Speicher des Rechners abgelegt werden.

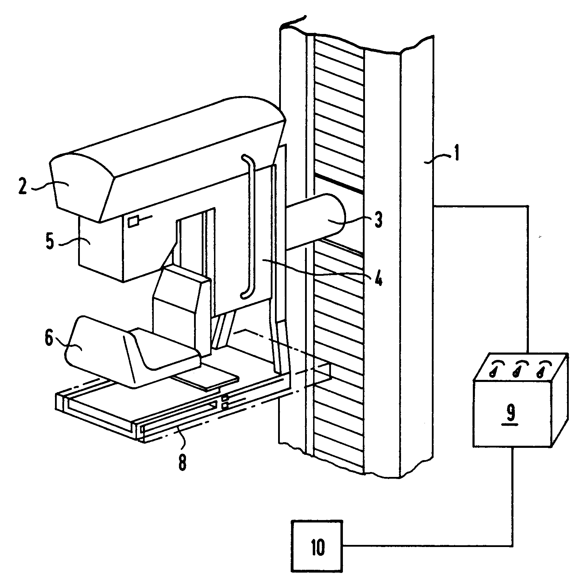

Die Erfindung ist nachfolgend anhand eines in der Zeichnung dargestellten Ausführungsbeispieles näher erläutert.

In der Zeichnung ist ein Stativ 1 dargestellt, mit dem eine Röntgenstrahlenquelle 2 mit Hilfe einer senkrecht zu deren Zentralstrahl liegenden Achse 3 höhenverstellbar verbunden ist. Die Achse 3 trägt die

2

Röntgenstrahlenquelle 2 mit Hilfe eines Blockes 4, an dem eine Primärstrahlenblende 5 angeordnet ist.

Der Block 4 trägt ferner eine motorisch in Richtung des Zentralstrahles der Röntgenstrahlenquelle 2 verstellbare Kompressionsplatte 6. Zur Auflage des Untersuchungsobjektes ist ein Aufnahmetisch 8 vorgesehen. Das Untersuchungsobjekt wird auf dem Aufnahmetisch 8, in den eine Röntgenfilmkassette eingeschoben werden kann, mit Hilfe der Kompressionsplatte 6 komprimiert.

Die Speisung und Steuerung des Röntgendiagnostikgerätes erfolgt mit Hilfe eines Bedienpultes 9, an dem die Aufnahmewerte eingestellt werden können. Dem Bedienpult 9 ist ein Rechner 10 zugeordnet, dem den jeweiligen Aufnahmewerten entsprechende elektrische Signale zugeführt werden und der daraus die mittlere Parenchymdosis nach folgender Formel berechnet:

$$\overline{D} = \frac{f \cdot J_E}{a \cdot (d - 2\, d_F)} \cdot \left[ e^{-a \cdot d_F} - e^{-a(d - d_F)} \right]$$

mit

$f = 1,03$ (Umrechnungsfaktor)
$d$ = Objektdicke in cm
$d_F$ = Dicke des Fettgewebes
$d_F$ = 0,5 cm für $d \leq 5,0$ cm
$d_F$ = 1,0 cm für $d > 5,0$ cm

$J_E = k \cdot (I \cdot t)$

$I$ = Röntgenröhrenstrom
$t$ = Aufnahmezeit
$k$ = Kalibrierfaktor

$$a = \frac{1}{d} \cdot \ln \frac{J_E}{J_A}$$

$J_A$ = Austrittsdosis

Die Kalibrierung der Röntgendiagnostikanlage kann mit Hilfe von Phantomen verschiedener Dicken, z.B. aus Plexiglas, beispielsweise gemäß folgender Tabelle erfolgen:

| Röhrenspannung | Objektdicke | äquivalente Gewebedicke | Eintrittsdosis | Austrittsdosis | k-Faktor |
|---|---|---|---|---|---|
| 28 kV | 3 cm | 3 cm | $\mu$Gy | $\mu$Gy | $\mu$Gy/mA |
| 30 kV | 4 cm | 3,1 - 5 cm | $\mu$Gy | $\mu$Gy | $\mu$Gy/mA |
| 32 kV | 5 cm | 3,1 - 7 cm | $\mu$Gy | $\mu$Gy | $\mu$Gy/mA |
| 34 kV | 6 cm | 7 cm | $\mu$Gy | $\mu$Gy | $\mu$Gy/mA |

Die Austrittsdosis ist dabei durch das verwendete Film-Folien-System bestimmt und so zu wählen, daß am Film eine mittlere Schwärzung von 1,5 erzielt wird.

Für verschiedene Aufnahmetechniken kann eine Korrektur der berechneten Eintrittsdosis nach folgenden Formeln erfolgen:

$$JE_{korr} = J_E \cdot \left( \frac{60 - d_P}{60 - d} \right)^2 \qquad \text{für Rastertechnik}$$

$$JE_{korr} = J_E \cdot \left( \frac{33.9 - d_P}{33.9 - d} \right)^2 \qquad \text{für Vergrößerungs-technik}$$

$$d_P, \; d \;\; \text{in cm}, \; d_P = \text{Phantomdicke}$$

**Patentansprüche**

**1.** Röntgendiagnostikanlage für Mammographieaufnahmen, die einen Rechner aufweist, **dadurch gekennzeichnet,** daß der Rechner (10) dazu angepaßt ist, aus den jeweiligen Aufnahmewerten entsprechenden, ihm zugeführten, elektrischen Signalen die mittlere Parenchymdosis (D) nach folgender Formel zu berechnen:

$$\overline{D} = \frac{f \cdot J_E}{a \cdot (d - 2 \, d_F)} \cdot \left[ e^{-a \cdot d_F} \quad -e^{-a(d - d_F)} \right]$$

mit
f = 1,03 (Umrechnungsfaktor)
d = Objektdicke in cm
$d_F$ = Dicke des Fettgewebes
$d_F$ = 0,5 cm für d ≤ 5,0 cm
$d_F$ = 1,0 cm für d > 5,0 cm

$J_E = k \cdot (I \cdot t)$

I = Röntgenröhrenstrom
t = Aufnahmezeit
k = Kalibrierfaktor

$$a = \frac{1}{d} \cdot \ln \frac{J_E}{J_A}$$

$J_A$ = Austrittsdosis

**2.** Röntgendiagnostikanlage für Mammographieaufnahmen nach Anspruch 1, **dadurch gekennzeichnet,** daß eine Korrektur der berechneten Eintrittsdosis nach folgenden Formeln erfolgt:

$$JE_{korr} = J_E \cdot \left( \frac{60 - d_P}{60 - d} \right)^2 \qquad \text{für Rastertechnik}$$

$$JE_{korr} = J_E \cdot \left( \frac{33.9 - d_P}{33.9 - d} \right)^2 \quad \text{für Vergrößerungs-technik}$$

$$d_P, \; d \;\; \text{in cm}, \;\; d_P = \text{Phantomdicke}$$

## Claims

1. X-ray diagnostic installation for mammography exposures having a computer, characterised in that the computer (10) is adapted to calculating from electric signals which correspond to the respective exposure values and which are supplied to it, the mean parenchyma dose (D) according to the following formula:

$$\overline{D} = \frac{f \cdot J_E}{a \cdot (d - 2\,d_F)} \cdot \left[ e^{-a \cdot d_F} \; -e^{-a(d - d_F)} \right]$$

with
f = 1.03 (conversion factor)
d = subject thickness in cm
$d_F$ = thickness of the fatty tissue
$d_F$ = 0.5 cm for d ≤ 5.0 cm
$d_F$ = 1.0 cm for d > 5.0 cm

$J_E = k \cdot (I \cdot t)$

I = X-ray tube current
t = exposure time
k = calibration factor

$$a = \frac{1}{d} \cdot \ln \frac{J_E}{J_A}$$

$J_A$ = exit dose.

2. X-ray diagnostic installation for mammography exposures according to claim 1, characterised in that the calculated entry dose is corrected according to the following formulae:

$$JE_{korr} = J_E \cdot \left( \frac{60 - d_p}{60 - d} \right)^2 \quad \text{for scan technology}$$

$$JE_{korr} = J_E \cdot \left( \frac{33.9 - d_p}{33.9 - d} \right)^2 \qquad \text{for enlargement technology}$$

$$d_p, \ d \ \text{in cm,} \ d_p = \text{phantom thickness.}$$

**Revendications**

1. Installation de radiodiagnostic pour la réalisation de clichés mammographiques, qui comporte un calculateur, caractérisée par le fait que le calculateur (10) est adapté de manière à calculer, à partir de signaux électriques qui correspondent aux valeurs respectives des clichés et lui sont envoyés, une dose moyenne de parenchyme (D) conformément à la formule suivante :

$$\overline{D} = \frac{f \cdot J_E}{a \cdot (d-2\,d_F)} \cdot \left[ e^{-a.d_F} - e^{-a(d-d_F)} \right]$$

avec
f = 1,03 (facteur de conversion)
d = épaisseur de l'objet en cm
$d_F$ = épaisseur du tissu adipeux
$d_F$ = 0,5 cm pour d $\leq$ 5,0 cm
$d_F$ = 1,0 cm pour d > 5,0 cm

$J_E = K \cdot (I \cdot t)$

I = courant du tube à rayons X
t = durée d'enregistrement
k = facteur de calibrage

$$a = \frac{1}{d} \cdot \ln \frac{J_E}{J_A}$$

$J_A$ = dose de sortie

2. Installation de radiodiagnostic pour la prise de clichés mammographiques suivant la revendication 1, caractérisée par le fait qu'une correction de la dose d'entrée calculée est effectuée conformément aux formules suivantes

$$JE_{korr} = J_E \cdot \left( \frac{60 - d_P}{60 - d} \right)^2 \qquad \text{pour la technique à trame}$$

$$JE_{korr} = J_E \cdot \left( \frac{33,9 - d_P}{33,9 - d} \right)^2 \qquad \text{pour la technique d'agrandissement}$$

$$d_P, \ d \ \text{en cm, et} \ d_P = \text{épaisseur fantôme.}$$